(19) Europäisches Patentamt
European Patent Office
Office européen des brevets

(11) **EP 3 763 283 B1**

(12) **FASCICULE DE BREVET EUROPEEN**

(45) Date de publication et mention
de la délivrance du brevet:
**16.03.2022 Bulletin 2022/11**

(21) Numéro de dépôt: **20184173.1**

(22) Date de dépôt: **06.07.2020**

(51) Classification Internationale des Brevets (IPC):
**A61B 5/024** *(2006.01)*    **A61B 5/0245** *(2006.01)*
**A61B 5/00** *(2006.01)*

(52) Classification Coopérative des Brevets (CPC):
**A61B 5/7221; A61B 5/02405;** A61B 5/02416;
A61B 5/0245

(54) **PROCÉDÉ D'ESTIMATION DE LA QUALITÉ D'UN SIGNAL DE RYTHME CARDIAQUE**

VERFAHREN ZUR EINSCHÄTZUNG DER QUALITÄT EINES HERZRHYTHMUS-SIGNALS

METHOD FOR ESTIMATING THE QUALITY OF A HEART RATE SIGNAL

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **10.07.2019 FR 1907758**

(43) Date de publication de la demande:
**13.01.2021 Bulletin 2021/02**

(73) Titulaires:
• **Commissariat à l'Energie Atomique et aux Energies
Alternatives
75015 Paris (FR)**
• **Université Grenoble Alpes
38400 Saint-Martin-d'Hères (FR)**

(72) Inventeurs:
• **VILA, Gaël**
**38054 GRENOBLE CEDEX 9 (FR)**
• **GODIN, Christelle**
**38054 GRENOBLE CEDEX 9 (FR)**
• **CAMPAGNE, Aurélie**
**38600 FONTAINE (FR)**
• **CHARBONNIER, Sylvie**
**38130 ECHIROLLES (FR)**

(74) Mandataire: **Cabinet Beaumont
4, Place Robert Schuman
B.P. 1529
38025 Grenoble Cedex 1 (FR)**

(56) Documents cités:
**FR-A1- 3 017 789    US-A1- 2011 224 565**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

Domaine technique

La présente description concerne de façon générale le domaine des systèmes utilisant des capteurs de rythme cardiaque, et vise plus particulièrement un procédé d'estimation de la qualité des signaux de rythme cardiaque fournis par de tels capteurs.

Technique antérieure

Le rythme cardiaque d'un sujet est un paramètre physiologique utilisé dans de nombreuses applications, par exemple des applications de contrôle du stress, des applications de contrôle et/ou de prévention de certaines maladies chroniques, ou encore des applications de contrôle de l'activité physique du sujet.

Dans certaines applications, le sujet est amené à porter un capteur de rythme cardiaque en continu pendant de longues périodes, par exemple tout au long de la journée et/ou de la nuit, en conditions ambulatoires. De nombreux capteurs adaptés à ces applications sont désormais disponibles dans le commerce.

Pour certaines applications, la qualité des signaux fournis par les capteurs existants n'est toutefois pas toujours suffisante. C'est notamment le cas des applications exploitant la variabilité à court-terme du rythme cardiaque, par exemple les applications de contrôle du stress. Dans de telles applications, quelques battements manqués peuvent suffire à fausser significativement les analyses. Le document FR 3 017 789 A1 décrit un procédé de filtration et de détermination de la qualité d'un signal R-R.

Résumé de l'invention

Un mode de réalisation prévoit un procédé d'estimation, au moyen d'un dispositif électronique de traitement, de la qualité d'un signal de rythme cardiaque fourni par un capteur de rythme cardiaque lors d'une phase d'acquisition de durée W, ledit signal comprenant une suite de N échantillons $IBI_i$ ayant chacun une valeur représentative d'une durée entre deux battements cardiaques successifs détectés par le capteur, avec N entier supérieur ou égal à 2 et i entier allant de 1 à N, le procédé comportant une étape de calcul, au moyen du dispositif électronique de traitement, d'un indicateur de carence L représentatif de la différence entre la durée W de la phase d'acquisition et la somme des valeurs des échantillons $IBI_i$ du signal.

Selon un mode de réalisation de la présente invention, l'indicateur de carence L est représentatif d'un pourcentage de battements cardiaques manqués par le capteur pendant la phase d'acquisition.

Selon un mode de réalisation de la présente invention, l'indicateur de carence L est défini par la formule suivante :

$$L = \frac{W - \sum_{i=1}^{N} IBI_i}{W}$$

Selon un mode de réalisation de la présente invention, la durée W de la phase d'acquisition est comprise entre 20 et 120 secondes.

Selon un mode de réalisation de la présente invention, le procédé comprend en outre une étape de comparaison de l'indicateur de carence L à un seuil TH prédéfini et une étape de décision, sur la base du résultat de la comparaison, de prendre en compte ou non le signal de rythme cardiaque.

Selon un mode de réalisation de la présente invention, lors de l'étape de décision, le signal de rythme cardiaque est pris en compte uniquement si l'indicateur de carence L est inférieur au seuil TH.

Selon un mode de réalisation de la présente invention, le seuil TH est défini comme suit :

$$TH = \frac{\tau}{100} + \frac{\min\limits_{i \in 1,...,N} IBI_i}{W}$$

où $\tau$ est une marge de tolérance comprise entre 0 et 100 définissant un pourcentage maximum toléré de battements cardiaques manqués par le capteur pendant la phase d'acquisition.

Selon un mode de réalisation de la présente invention, la marge $\tau$ est comprise entre 0 et 20.

Un autre mode de réalisation prévoit un système comportant un capteur de rythme cardiaque et un dispositif électronique de traitement, le dispositif électronique de traitement étant configuré pour mettre en oeuvre un procédé d'estimation de la qualité d'un signal de rythme cardiaque fourni par le capteur tel que défini ci-dessus.

EP 3 763 283 B1

## Brève description des dessins

Ces caractéristiques et leurs avantages, ainsi que d'autres, seront exposés en détail dans la description suivante de modes de réalisation particuliers faite à titre non limitatif en relation avec les figures jointes parmi lesquelles :

la figure 1 représente de façon schématique, sous forme de blocs, un exemple d'un système comportant un capteur de rythme cardiaque et un dispositif électronique de traitement adapté à mettre en oeuvre un procédé d'estimation de la qualité d'un signal de rythme cardiaque selon un mode de réalisation ;

la figure 2 est un diagramme représentant, à titre d'illustration, un exemple d'un signal de rythme cardiaque ; et

la figure 3 représente de façon schématique, sous forme de blocs, un exemple d'un procédé d'estimation de la qualité d'un signal de rythme cardiaque selon un mode de réalisation.

## Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, la réalisation d'un capteur de rythme cardiaque adapté à fournir les signaux de rythme cardiaque traités par le procédé d'estimation de qualité de la présente demande n'a pas été détaillée, les modes de réalisation décrits étant compatibles avec tous ou la plupart des capteurs de rythme cardiaque connus, ou la réalisation d'un tel capteur étant à la portée de l'homme du métier à partir des indications de la présente description. De plus, la réalisation d'un dispositif électronique de traitement adapté à mettre en oeuvre le procédé décrit ci-après d'estimation de la qualité d'un signal de rythme cardiaque n'a pas été détaillée, la réalisation d'un tel dispositif étant à la portée de l'homme du métier à partir des indications de la présente description. En outre, les applications susceptibles d'exploiter les signaux de rythme cardiaque analysés par le procédé de la présente demande n'ont pas été détaillées, les modes de réalisation décrits étant compatibles avec toutes ou la plupart des applications pouvant tirer profit de la mise à disposition d'un indicateur représentatif de la qualité d'un signal de rythme cardiaque fourni par un capteur de rythme cardiaque.

Sauf précision contraire, lorsque l'on fait référence à deux éléments connectés entre eux, cela signifie directement connectés sans éléments intermédiaires autres que des conducteurs, et lorsque l'on fait référence à deux éléments reliés ou couplés entre eux, cela signifie que ces deux éléments peuvent être connectés ou être reliés ou couplés par l'intermédiaire d'un ou plusieurs autres éléments.

Sauf précision contraire, les expressions "environ", "approximativement", "sensiblement", et "de l'ordre de" signifient à 10 % près, de préférence à 5 % près.

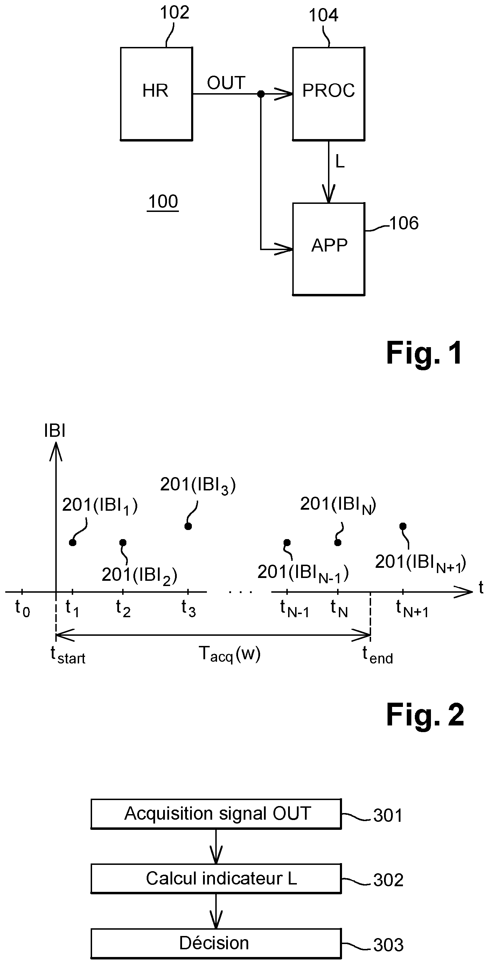

La figure 1 représente de façon schématique, sous forme de blocs, un exemple d'un système 100 comportant un capteur de rythme cardiaque 102 (HR) et un dispositif électronique de traitement 104 (PROC) configuré pour mettre en oeuvre un procédé d'estimation de la qualité d'un signal de rythme cardiaque fourni par le capteur 102.

Le capteur 102 peut être un capteur de type électrocardiographe, mesurant l'activité électrique du coeur au moyen d'électrodes placées en contact avec la surface de la peau du sujet. A titre de variante, le capteur 102 peut être un capteur de type photopléthysmographe, mesurant les variations d'un signal lumineux, par exemple un signal infrarouge, sur le trajet duquel est placé un vaisseau sanguin du sujet (par exemple au niveau d'un poignet de l'utilisateur dans le cas d'un capteur de type bracelet). Plus généralement, les modes de réalisation décrits s'appliquent à tout type de capteur adapté à mesurer un signal représentatif des battements de coeur du sujet.

On considère ici plus particulièrement un capteur fournissant un signal de sortie OUT sous la forme d'une suite d'échantillons ayant chacun une valeur représentative d'une période entre deux battements de coeur successifs détectés par le capteur. On peut alors parler de signal de rythme cardiaque instantané dans la mesure où chaque échantillon est représentatif de l'intervalle inter-battement courant et donc de la fréquence cardiaque courante (instantanée) du sujet.

Dans l'exemple de la figure 1, le signal OUT est transmis à un dispositif électronique applicatif 106 (APP) configuré pour mettre en oeuvre un procédé exploitant le signal OUT, par exemple un procédé de contrôle du stress, un procédé de contrôle et/ou de prévention de certaines maladies chroniques, ou encore un procédé de contrôle de l'activité physique du sujet. La liaison entre le capteur 102 et le dispositif 106 est par exemple une liaison filaire.

Pour générer le signal OUT, le capteur 102 comprend un circuit interne de traitement, non détaillé sur la figure. Le circuit interne de traitement génère le signal OUT à partir d'un signal brut analogique fourni par un élément d'acquisition (non détaillé) du capteur. En pratique, selon le type de capteur utilisé et en fonction des conditions d'utilisation du capteur, par exemple en cas de positionnement imparfait du capteur, certaines portions du signal analogique fourni par l'élément d'acquisition du capteur peuvent être trop bruitées pour réaliser une détection fiable des battements cardiaques. Ces portions peuvent être identifiées par le dispositif interne de traitement du capteur et ne sont alors pas prises en compte pour générer le signal OUT. De plus, certaines valeurs d'intervalle inter-battement calculées peuvent être jugées aberrantes par le dispositif interne de traitement du capteur, par exemple si elles ne satisfont pas un critère de pertinence prédéterminé, et ne sont alors pas transmises dans le signal de sortie OUT du capteur. Ainsi, certains battements de coeur du sujet ne sont pas pris en compte pour la génération du signal OUT. Autrement dit, le signal OUT fourni par le capteur 102 est un signal débarrassé ou nettoyé d'éventuelles valeurs aberrantes. Le procédé mis en oeuvre par le capteur 102 pour générer, à partir du signal brut, un signal de sortie OUT débarrassé de valeurs aberrantes, ne sera pas détaillé, les modes de réalisation décrits étant compatibles avec toutes ou la plupart des méthodes connues de nettoyage d'un signal brut fourni par un capteur de rythme cardiaque.

Selon le type d'application mis en oeuvre par le dispositif 106, les battements manquants peuvent conduire à fausser significativement les résultats obtenus. En particulier, les carences de battements sont tout particulièrement problématiques pour les applications exploitant la variabilité à court-terme du rythme cardiaque, par exemple les applications de contrôle du stress.

Pour cette raison, dans le système de la figure 1, le signal de sortie OUT du capteur 102 est en outre transmis au dispositif 104 qui met en oeuvre un procédé d'estimation de la qualité du signal OUT. Plus particulièrement, le dispositif 104 calcule un indicateur L de la qualité du signal OUT. L'indicateur L est transmis au dispositif applicatif 106 qui, sur la base de cet indicateur, détermine si le signal OUT peut ou non être exploité par l'application. La liaison entre le capteur 102 et le dispositif de traitement 104 est par exemple une liaison filaire. La liaison entre le dispositif de traitement 104 et le dispositif applicatif 106 peut également être une liaison filaire.

Le dispositif de traitement 104 peut comprendre un microprocesseur, ou tout autre circuit de traitement adapté à mettre en oeuvre le procédé de calcul de l'indicateur L décrit ci-après. A titre d'exemple, le dispositif applicatif 106 et le circuit de traitement 104 comprennent des éléments communs, par exemple un même microprocesseur.

La figure 2 est un diagramme représentant, à titre d'illustration, un exemple d'un signal de rythme cardiaque. Plus particulièrement, le diagramme de la figure 2 représente l'évolution, en fonction du temps t (en abscisse), de l'intervalle inter-battement IBI (en ordonnée) du sujet.

Sur la figure 2, des instants $t_0$, $t_1$, $t_2$, $t_3$, ... $t_{N-1}$, $t_N$, $t_{N+1}$ ont été représentés sur l'axe des abscisses, correspondant respectivement à des instants d'occurrences de battements cardiaques successifs du sujet. Pour chacun des instants $t_i$, avec i entier allant de 1 à N+1, on a représenté sur la figure 2 un point 201 ayant pour abscisse l'instant $t_i$, et pour ordonnée une valeur $IBI_i = t_i - t_{i-1}$ correspondant à l'intervalle de temps écoulé entre les battements cardiaques des instants $t_{i-1}$ et $t_i$.

Le signal de sortie OUT du capteur 102 est par exemple constitué par la suite des valeurs $IBI_1$, $IBI_2$, $IBI_3$, ..., $IBI_{N-1}$, $IBI_N$, $IBI_{N+1}$.

La figure 3 représente de façon schématique, sous forme de blocs, un exemple d'un procédé d'estimation de la qualité du signal de rythme cardiaque OUT fourni par le capteur 102, mis en oeuvre par le dispositif de traitement 104.

Le procédé de la figure 3 comprend une étape 301 d'acquisition du signal OUT pendant une phase d'acquisition $T_{acq}$ de durée W, allant d'un instant $t_{start}$ à un instant $t_{end}$. La durée W de la phase d'acquisition $T_{acq}$ est par exemple comprise entre 20 et 120 secondes. Les modes de réalisation décrits ne se limitent toutefois pas à ce cas particulier.

Le signal OUT acquis pendant la phase d'acquisition $T_{acq}$ est constitué de N échantillons successifs $IBI_1$, ..., $IBI_N$, représentatifs chacun d'une durée entre deux battements cardiaques successifs du sujet.

Lorsque tous les battements de coeur du sujet sont effectivement pris en compte au sein du capteur 102 pour générer le signal OUT, la somme des valeurs des N échantillons $IBI_1$, ..., $IBI_N$ acquis pendant la phase d'acquisition $T_{acq}$ est proche de la durée W de la phase d'acquisition $T_{acq}$. Plus particulièrement, en considérant que l'instant $t_{start}$ de début de la phase d'acquisition $T_{acq}$ peut être compris entre deux battements de coeur successifs du sujet, entre les battements $t_0$ et $t_1$ dans l'exemple représenté, et que l'instant $t_{end}$ de fin de la phase d'acquisition $T_{acq}$ peut être compris entre deux battements de coeur successifs du sujet, entre les battements $t_N$ et $t_{N+1}$ dans l'exemple représenté, la relation suivante est respectée :

[Math. 1]

$$\sum_{i=1}^{N} IBI_i - IBI_1 < W < \sum_{i=1}^{N} IBI_i + IBI_{N+1}$$

Si en revanche certains intervalles inter-battement du patient n'ont pas été pris en compte par le capteur 102 et n'ont par conséquent pas été transmis dans le signal OUT, la somme des valeurs des échantillons $IBI_1$, ..., $IBI_N$ acquis pendant la phase d'acquisition $T_{acq}$ peut être sensiblement inférieure à la durée W de la phase d'acquisition $T_{acq}$.

Selon un aspect des modes de réalisation décrits, on prévoit, lors d'une étape 302 postérieure à l'étape 301, de calculer un indicateur L représentatif de la différence entre la durée W de la phase d'acquisition et la somme des valeurs des échantillons du signal. Cet indicateur, aussi appelé indicateur de carence, est représentatif de la proportion de périodes inter-battement non prises en compte par le capteur 102, et est utilisé comme indicateur de la qualité du signal de sortie OUT du capteur 102.

L'indicateur L est par exemple défini comme suit :

[Math. 2]

$$L = \frac{W - \sum_{i=1}^{N} IBI_i}{W}$$

L'indicateur L est ainsi représentatif du pourcentage de battements non pris en compte dans la fenêtre d'acquisition $T_{acq}$.

Une autre façon d'exprimer l'indicateur L est de considérer un nombre théorique $N_{th}$ d'échantillons qui aurait dû être acquis pendant la phase d'acquisition $T_{acq}$, défini comme suit :

[Math. 3]

$$N_{th} = \frac{W}{\mu}$$

où $\mu$ désigne la moyenne des valeurs des échantillons $IBI_1, ... IBI_N$, soit :

[Math. 4]

$$N_{th} = \frac{W * N}{\sum_{i=1}^{N} IBI_i}$$

On a alors :

[Math. 5]

$$L = \frac{N_{th} - N}{N_{th}}$$

Sur la figure 3, on a en outre représenté une étape 303, postérieure à l'étape 302, de décision, sur la base de l'indicateur L, de tenir compte ou non du signal OUT acquis pendant la phase d'acquisition $T_{acq}$. L'étape de décision 303 peut être mise en oeuvre par le dispositif applicatif 106 lui-même, ou par le dispositif de traitement 104. Dans ce dernier cas, seule une valeur binaire représentative du résultat de la décision peut être transmise au dispositif applicatif 106.

L'étape de décision consiste par exemple à comparer l'indicateur L à un seuil TH prédéfini, et à tenir compte du signal OUT acquis pendant la phase d'acquisition $T_{acq}$ uniquement si l'indicateur L est inférieur au seuil TH.

Le seuil TH est par exemple défini comme suit :

[Math. 6]

$$TH = \frac{\tau}{100} + \frac{\min_{i \in 1,...,N} IBI_i}{W}$$

où $\tau$ est une marge de tolérance comprise entre 0 et 100, par exemple entre 0 et 20, définissant le pourcentage maximal toléré de battements manqués pendant la phase d'acquisition $T_{acq}$.

A titre d'exemple, la fenêtre temporelle d'acquisition $T_{acq}$ de durée W est une fenêtre glissante, le procédé de la figure 3 étant réitéré à chaque fois qu'un nouvel échantillon du signal OUT est fourni par le capteur 102. Ceci permet de détecter et de prendre en compte toutes les portions du signal OUT de durée W répondant au critère de qualité défini à l'étape 303.

Divers modes de réalisation et variantes ont été décrits. L'homme de l'art comprendra que certaines caractéristiques de ces divers modes de réalisation et variantes pourraient être combinées, et d'autres variantes apparaîtront à l'homme de l'art. En particulier, les modes de réalisation décrits ne se limitent pas aux exemples de paramètres numériques décrits ci-dessus.

Enfin, la mise en oeuvre pratique des modes de réalisation et variantes décrits est à la portée de l'homme du métier à partir des indications fonctionnelles données ci-dessus.

## Revendications

1. Procédé d'estimation, au moyen d'un dispositif électronique de traitement (104), de la qualité d'un signal de rythme cardiaque (OUT) fourni par un capteur de rythme cardiaque (102) lors d'une phase d'acquisition ($T_{acq}$) de durée W, ledit signal (OUT) étant fourni débarrassé d'éventuelles valeurs aberrantes et comprenant une suite de N échantillons $IBI_i$ ayant chacun une valeur représentative d'une durée entre deux battements cardiaques successifs détectés par le capteur (102), avec N entier supérieur ou égal à 2 et i entier allant de 1 à N, le procédé comportant une étape (302) de calcul, au moyen du dispositif électronique de traitement (104), d'un rapport ou d'une différence entre la durée W de la phase d'acquisition ($T_{acq}$) et la somme des valeurs des échantillons $IBI_i$ du signal (OUT), et une étape de fourniture d'un indicateur de carence L fonction de ce rapport ou de cette différence.

2. Procédé selon la revendication 1, dans lequel l'indicateur de carence L est représentatif d'un pourcentage de battements cardiaques manqués par le capteur (102) pendant la phase d'acquisition (Tacq).

3. Procédé selon la revendication 1 ou 2, dans lequel l'indicateur de carence L est défini par la formule suivante :

$$L = \frac{W - \sum_{i=1}^{N} IBI_i}{W}$$

4. Procédé selon l'une quelconque des revendications 1 à 3, dans lequel la durée W de la phase d'acquisition ($T_{acq}$) est comprise entre 20 et 120 secondes.

5. Procédé selon l'une quelconque des revendications 1 à 4, comprenant en outre une étape de comparaison de l'indicateur de carence L à un seuil TH prédéfini et une étape de décision, sur la base du résultat de la comparaison, de prendre en compte ou non le signal de rythme cardiaque (OUT).

6. Procédé selon la revendication 5, dans lequel, lors de l'étape de décision, le signal de rythme cardiaque (OUT) est pris en compte uniquement si l'indicateur de carence L est inférieur au seuil TH.

7. Procédé selon la revendication 5 ou 6, dans lequel le seuil TH est défini comme suit :

$$TH = \frac{\tau}{100} + \frac{\min_{i \in 1,...,N} IBI_i}{W}$$

où $\tau$ est une marge de tolérance comprise entre 0 et 100 définissant un pourcentage maximum toléré de battements cardiaques manqués par le capteur (102) pendant la phase d'acquisition ($T_{acq}$).

8. Procédé selon la revendication 7, dans lequel la marge $\tau$ est comprise entre 0 et 20.

9. Système comportant un capteur de rythme cardiaque (102) et un dispositif électronique de traitement (104), le dispositif électronique de traitement (104) étant configuré pour mettre en oeuvre un procédé d'estimation de la qualité d'un signal de rythme cardiaque (OUT) fourni par le capteur (102) selon l'une quelconque des revendications 1 à 8.

**Patentansprüche**

1. Ein Verfahren zur Schätzung, mittels einer elektronischen Verarbeitungsvorrichtung (104), der Qualität eines von einem Herzfrequenzsensor (102) während einer Erfassungsphase ($T_{acq}$) der Dauer W gelieferten Herzfrequenzsignals (OUT), wobei das Signal (OUT) von möglichen Ausreißern befreit ist und eine Folge von N Abtastwerten $IBI_i$ aufweist, die jeweils einen Wert aufweisen, der für eine Dauer zwischen zwei aufeinanderfolgenden, vom Sensor (102) erfassten Herzschlägen repräsentativ ist, wobei N eine ganze Zahl größer oder gleich 2 ist und i eine ganze Zahl zwischen 1 und N ist, wobei das Verfahren einen Schritt (302) der Berechnung, durch die elektronische Verarbeitungsvorrichtung (104), eines Verhältnisses oder einer Differenz zwischen der Dauer W der Erfassungsphase ($T_{acq}$) und der Summe der Werte der Abtastwerte $IBI_i$ des Signals (OUT) und einen Schritt der Ausgabe eines Standardindikators L aufweist, der eine Funktion von diesem Verhältnis oder dieser Differenz ist.

2. Verfahren nach Anspruch 1, wobei der Standardindikator L repräsentativ für einen Prozentsatz der vom Sensor (102) während der Erfassungsphase ($T_{acq}$) verpassten Herzschläge ist.

3. Verfahren nach Anspruch 1 oder 2, wobei der Standardindikator L durch die folgende Formel definiert ist:

$$L = \frac{W - \sum_{i=1}^{N} IBI_i}{W}$$

4. Verfahren nach einem der Ansprüche 1 bis 3, wobei die Dauer W der Erfassungsphase ($T_{acq}$) im Bereich von 20 bis 120 Sekunden liegt.

5. Verfahren nach einem der Ansprüche 1 bis 4, das ferner einen Schritt des Vergleichs des Standardindikators L mit einem vordefinierten Schwellenwert TH und einen Schritt der Entscheidung auf der Grundlage des Ergebnisses des Vergleichs aufweist, das Herzfrequenzsignal (OUT) zu berücksichtigen oder nicht.

6. Verfahren nach Anspruch 5, wobei während des Entscheidungsschritts das Herzfrequenzsignal (OUT) nur berücksichtigt wird, wenn der Standardindikator L kleiner als der Schwellenwert TH ist.

7. Verfahren nach Anspruch 5 oder 6, wobei der Schwellenwert TH wie folgt definiert ist:

$$TH = \frac{\tau}{100} + \frac{\min_{i=1,\dots,N} IBI_i}{W}$$

wobei $\tau$ eine Toleranzspanne zwischen 0 und 100 ist, die einen maximal tolerierten Prozentsatz der vom Sensor (102) während der Erfassungsphase ($T_{acq}$) verpassten Herzschläge definiert.

8. Verfahren nach Anspruch 7, wobei die Spanne $\tau$ im Bereich von 0 bis 20 liegt.

9. Ein System mit einem Herzfrequenzsensor (102) und einer elektronischen Verarbeitungsvorrichtung (104), wobei die elektronische Verarbeitungsvorrichtung (104) so konfiguriert ist, dass sie ein Verfahren zur Schätzung der Qualität eines vom Sensor (102) gelieferten Herzfrequenzsignals (OUT) nach einem der Ansprüche 1 bis 8 implementiert.

**Claims**

1. A method of estimation, by means of an electronic processing device (104), of the quality of a heart rate signal (OUT) delivered by a heart rate sensor (102) during an acquisition phase ($T_{acq}$) of duration W, said signal (OUT) being delivered rid of possible outliers and comprising a sequence of N samples $IBI_i$ each having a value representative of a duration between two successive heartbeats detected by the sensor (102), N being an integer greater than or equal to 2 and i being an integer in the range from 1 to N, the method comprising a step (302) of calculation, by means of the electronic processing device (104), of a ratio or a difference between duration W of the acquisition phase ($T_{acq}$) and the sum of the values of the samples $IBI_i$ of the signal (OUT), and a step of delivery of a default indicator L which is a function of this ratio or of this difference.

2. The method according to claim 1, wherein the default indicator L is representative of a percentage of heartbeats missed by the sensor (102) during the acquisition phase ($T_{acq}$).

3. The method of claim 1 or 2, wherein the default indicator L is defined by the following formula:

$$L = \frac{W - \sum_{i=1}^{N} IBI_i}{W}$$

4. The method of any of claims 1 to 3, wherein the duration W of the acquisition phase ($T_{acq}$) is in the range from 20 to 120 seconds.

5. The method according to any of claims 1 to 4, further comprising a step of comparison of default indicator L with a predefined threshold TH and a step of decision, based on the result of the comparison, to take into account or not the heart rate signal (OUT).

6. The method according to claim 5, wherein, during the decision step, the heart rate signal (OUT) is taken into account only if default indicator L is smaller than threshold TH.

7. The method according to claim 5 or 6, wherein threshold TH is defined as follows:

$$TH = \frac{\tau}{100} + \frac{\min_{i \in 1,\ldots,N} IBI_i}{W}$$

where $\tau$ is a tolerance margin between 0 and 100 defining a maximum tolerated percentage of heartbeats missed by the sensor (102) during the acquisition phase ($T_{acq}$).

8. The method according to claim 7, wherein margin $\tau$ is in the range from 0 to 20.

9. A system comprising a heart rate sensor (102) and an electronic processing device (104), the electronic processing device (104) being configured to implement a method of estimation of the quality of a heart rate signal (OUT) delivered by the sensor (102) according to any of claims 1 to 8.

102                                   104

┌──────────┐        OUT        ┌──────────┐
│          │      ●─────────→  │          │
│    HR    │──────●            │   PROC   │
│          │      │            │          │
└──────────┘      │            └──────────┘
                  │                  │
                  │                  │ L
     100          │                  ↓
                  │            ┌──────────┐
                  │            │          │ ─ 106
                  └──────────→ │   APP    │
                               │          │
                               └──────────┘

## Fig. 1

IBI

201(IBI₃)
201(IBI₁)                         201(IBI_N)

201(IBI₂)          201(IBI_{N-1})      201(IBI_{N+1})

            . . .

t₀  t₁  t₂  t₃         t_{N-1}  t_N      t_{N+1}     t

t_start                              t_end

T_{acq}(w)

## Fig. 2

┌────────────────────────────┐
│   Acquisition signal OUT    │ ── 301
└────────────────────────────┘
              ↓
┌────────────────────────────┐
│     Calcul indicateur L     │ ── 302
└────────────────────────────┘
              ↓
┌────────────────────────────┐
│         Décision           │ ── 303
└────────────────────────────┘

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3017789 A1